# FASCICULE DE BREVET EUROPEEN

(11) **EP 3 724 345 B1**
(45) Date de publication et mention de la délivrance du brevet: **10.12.2025**
(21) Numéro de dépôt: 18819182.9
(22) Date de dépôt: 12.11.2018
(51) Int. Cl.: C12P 19/30, A23L 5/20, A23L 27/23, A23L 31/15

(54) **EXTRAIT DE LEVURE RICHE EN RIBONUCLÉOTIDES ET SON UTILISATION POUR LE MASQUAGE DE GOÛTS INDÉSIRABLES ET DE NOTES AROMATIQUES INDÉSIRABLES**
HEFEEXTRAKT REICH AN RIBONUCLEOTIDEN UND SEINE VERWENDUNG ZUR MASKIERUNG UNERWÜNSCHTER GESCHMACKS UND DUFTNOTEN
YEAST EXTRACT RICH IN RIBONUCLEOTIDES AND ITS USE FOR MASKING UNDESIRABLE TASTE AND AROMATIC NOTES

(30) Priorité: 13.12.2017 FR 1762074
(43) Date de publication de la demande: 21.10.2020
(73) Titulaire: Lesaffre et Compagnie, 75001 Paris (FR)
(72) Inventeur: JOLIVET, Hélène, 92300 Levallois Perret (FR); MENIN, Rudy, 94600 CHOISY LE ROI (FR); THOMAS, Antoine, 94160 Saint Mandé (FR)
(74) Mandataire: Plasseraud IP
(86) Numéro de dépôt international: PCT/FR2018/052808
(87) Numéro de publication internationale: WO 2019/115894

(56) Documents cités:
- WO-A2-2004/067758
- FR-A1- 2 922 728
- JP-A- 2002 101 846
- "Automatic translation of JP2002101846. METHOD FOR PRODUCING YEAST EXTRACT HIGHLY CONTAINING 5'-NUCLEOTIDE - (KOJK ) KOHJIN CO LTD", 28 septembre 2000 (2000-09-28) XP005518485

## Description

L'invention concerne un nouvel extrait de levure riche en 5'-ribonucléotides, son utilisation pour le masquage de goûts indésirables et de notes aromatiques indésirables, ainsi qu'un procédé de masquage goût indésirables et de notes aromatiques indésirables au sein d'un produit.

La perception du goût d'un aliment ou d'une substance est le fruit d'une multitude de mécanismes et se trouve être la résultante de la saveur, de l'arôme et des sensations trigéminales. Le goût d'un aliment est également désigné par le terme de flaveur. Lors de la mise en bouche d'un aliment, la mastication entraine la libération des constituants volatils et non volatils, chacun de ces constituants ayant des conséquences et des effets variés sur la perception du goût.

Les constituants non volatils, par l'intermédiaire des cellules réceptrices du système gustatif, sont responsables de la saveur. Il existe uniquement cinq saveurs fondamentales, à savoir l'acide, l'amer, le sucré, le salé et l'umami.

Les constituants volatils sont quant à eux détectés par l'intermédiaire des cellules réceptrices du système olfactif, on parle alors de voie rétronasale, et sont responsables des arômes. A l'inverse des saveurs, il existe une grande variété d'arômes, lesdits arômes pouvant être la résultante de plusieurs molécules.

Enfin, conjointement, les constituants volatils et non volatils sont détectés par les terminaisons nerveuses du nerf trijumeau au sein des cavités buccales ou nasales. Cette détection entraine une grande diversité de sensations telles que le piquant, l'astringent, le chaud ou le froid.

Ainsi, la perception du goût des aliments ou des substances, de par la saveur, les arômes et les sensations trigéminales, est un phénomène complexe.

Malgré cette complexité, il est parfois nécessaire pour certaines applications, notamment alimentaires ou pharmaceutiques, de pouvoir masquer des saveurs ou les notes de certains arômes.

De ce fait, depuis de nombreuses années les industriels développent des solutions permettant d'améliorer ou de masquer certaines saveurs ou arômes dans des produits destinés à être ingérés. Effectivement, certaines saveurs nécessitent de pouvoir être améliorées sans qu'il faille pour autant augmenter les quantités des composés non volatils responsables de ces saveurs, alors que d'autres devraient au contraire pouvoir être masquées, il s'agit là notamment des goûts indésirables.

Le document JP2002101846A décrit un procédé de préparation d'un extrait de levures riche en 5'-ribonucléotides ne présentant pas d'odeur de levure sans avoir recours à des souches de levures présentant des taux particulièrement élevés d'ARN.

Concernant l'amélioration des saveurs, un bon exemple est celui de la saveur sucrée pour laquelle il ne serait pas acceptable au regard des conséquences sur la santé d'augmenter les quantités en sucre de l'aliment pour en améliorer la perception. C'est ainsi que des solutions alternatives se sont développées, pour en améliorer la perception sans toutefois augmenter le taux de sucre du produit.

Par exemple, le document EP 1 080 645 décrit l'utilisation d'un agent pour améliorer la saveur sucrée dans les produits alimentaires contenant des édulcorants. Particulièrement, l'agent est un extrait de levure contenant 1 à 15% en poids de 5'-ionosinate de sodium et/ou 5'-adénylate de sodium, 1 à 15% en poids de 5'-guanylate de sodium, 1 à 15% en poids de 5'-uridylate de sodium, 1 à 15% en poids de 5'-cytidylate de sodium et 1 à 20% en poids de glutamate de sodium, les pourcentages en poids étant rapportés au poids de l'extrait de levure. Un inconvénient de ces extraits de levure réside notamment dans le fait qu'ils permettent uniquement de potentialiser la saveur sucré, et n'offrent donc pas la possibilité d'effectuer un masquage de goûts et/ou de notes aromatiques particulières dans des produits pour lesquels lesdits goûts ou lesdites notes sont délétères.

Certains constituants utilisés pour la fabrication ou la conservation des produits alimentaires ne sont pas nécessairement neutres et laissent ainsi en bouche un goût indésirable ou des saveurs résiduelles. De même, certains produits présentent en tant que tels des saveurs dont il est parfois nécessaire d'atténuer l'intensité. D'une manière intéressante, les extraits de levure peuvent également être utilisés pour réaliser un masquage de goûts indésirables ou de notes aromatiques indésirables.

Le document WO2003/063613 décrit l'utilisation d'un extrait de levure dans une composition à base d'édulcorant artificiel pour masquer les notes d'édulcorant. L'extrait de levure comprend des acides aminés libres et des 5'-ribonucléotides selon un ratio acides aminés libres/5'-ribonucléotides inférieur à 3,5. Le masquage des notes d'édulcorant est obtenu avec deux extraits de levure particuliers, un premier comprenant 13 % en poids de 5'-ribonucléotides dont 6,5 % en poids de 5'-IMP et 5'-GMP et un second comprenant 8,5 % en poids de 5'-ribonucléotides dont 4,3 % en poids de 5'-IMP et 5'-GMP. Bien que cet extrait puisse masquer une note, il ne s'agit que d'une note d'édulcorant et il serait avantageux de pouvoir disposer d'autres masquages.

Le document WO2005/0067734 décrit un procédé de préparation d'une composition pouvant être utilisée pour augmenter et/ou améliorer la saveur et les arômes des aliments, mais également pour masquer la saveur d'amertume engendrée par les édulcorants artificiels. La composition comprend des extraits de levure présentant 15 % à 55 % en poids de 5'-ribonucléotides. Les pourcentages étant donnés par rapport au poids de matières sèches sans NaCl de la composition. La description de ce document précise également que la quantité de guanosine 5'-monophosphate (5'-GMP) dans la composition est de préférence plus grande que la somme de la quantité d'adénosine 5'-monophosphate (5'-AMP) et de inosine 5'-monophosphate (5'-IMP) car le 5'-GMP est plus efficace pour améliorer la perception du goût que le 5'-IMP.

Les extraits de levure peuvent également être utilisés pour réaliser un masquage de notes métalliques de certains substituts de sel. Par exemple, le document WO2008/0068155 décrit l'utilisation d'extraits de levure comprenant au moins 30% en poids de 5'-ribonucléotides pour masquer les notes métalliques engendrées par les substituts de sels dans les produits céréaliers. Cet effet est obtenu avec des extraits de levure riches en 5'-ribonucléotides comprenant particulièrement au moins 20 % en poids de 5'-GMP et 5'-IMP. Les pourcentages sont donnés par rapport au poids de matières sèches sans NaCl de la composition.

Concernant les applications alimentaires, les extraits de levure peuvent donc aussi bien être utilisés pour rehausser des saveurs présentes dans les produits que pour masquer des notes indésirables. Cette dissemblance dans les applications s'explique notamment par la complexité et la diversité des composés contenus dans les extraits de levure rendant ainsi particulièrement difficile l'obtention de nouveaux extraits aux propriétés spécifiques. De même, comme développé précédemment, la perception du goût est régie par des mécanismes complexes impliquant plusieurs systèmes sensoriels et une grande variété de composés. Tout ceci explique probablement que pour masquer des goûts indésirables et des notes aromatiques indésirables, les industriels développent uniquement des extraits de levure ne permettant de réaliser qu'un seul type de masquage.

Ainsi, il existe un besoin de disposer de nouveaux extraits de levure capables de de réaliser plusieurs masquages sans qu'il soit nécessaire de modifier leur composition et sans génération d'un goût indésirable dans les produits au sein desquels ils sont incorporés. En effet, la flaveur d'un produit est le résultat d'une subtile équation. Ainsi, effectuer plusieurs masquages sans pour autant dénaturer la perception du goût en bouche avec un seul et même extrait était loin d'être évident.

Il est donc du mérite de la Demanderesse d'avoir réussi, au fruit de nombreuses recherches, à mettre au point un extrait de levure particulier, ledit extrait de levure présentant des propriétés combinées de masquage de goût indésirable et de notes aromatiques indésirables jusqu'alors jamais atteintes. Les extraits de levure mis au point trouvent ainsi une application toute particulière dans la fabrication de produits alimentaires, pharmaceutiques, nutraceutiques ou des préparations aromatiques.

La présente invention concerne donc un nouvel extrait de levure comprenant de 25 % à 55 % en poids de 5'-ribonucléotides, dont 5 % à 20 % en poids d'adénosine 5'-monophosphate (5'-AMP) et 5 % à 20 % en poids de guanosine 5'-monophosphate (5'-GMP) selon un ratio 5'-AMP/5'GMP allant de 0,85 à 1,25. Les pourcentages en poids étant exprimés par rapport au poids de matières sèches de l'extrait de levure et sont calculés en tenant compte de la forme disodique heptahydratée des 5'-ribonucléotides. L'invention concerne également l'utilisation d'un tel extrait de levure pour le masquage des saveurs amère et acide et des notes aromatiques indésirables d'édulcorants, de protéines, et métalliques dans un produit.

Pour l'ensemble de la description, et à des fins de clarification, les pourcentages en poids sont toujours exprimés par rapport au poids de matières sèches de l'extrait de levure, sauf lorsque le contexte permettra d'identifier le contraire sans aucune ambiguïté.

Au sens de la présente invention, le masquage d'un goût consiste à diminuer ou à complètement supprimer la perception d'une saveur au sein d'un produit, tandis que le masquage de note indésirable (en anglais « off-note ») consiste à diminuer ou complètement supprimer la perception d'un arôme ou d'une note aromatique.

L'extrait de levure selon l'invention est particulièrement remarquable en ce qu'il permet de masquer à la fois les saveurs amère et acide et les notes aromatiques indésirables d'édulcorants, de protéines et métalliques dans des produits sans pour autant dénaturer la perception globale du goût desdits produits. En effet, l'extrait de levure utilisé selon l'invention n'engendre notamment pas les notes de bouillon, les notes aromatiques de levure ou la saveur umami (regroupés sous l'expression anglaise « yeasty taste ») généralement caractéristiques de ces extraits.

Au sens de la présente invention, le terme produit désigne aussi bien un produit alimentaire, pharmaceutique, ou nutraceutique que des préparations aromatiques pouvant être utilisées dans des applications alimentaires, pharmaceutiques ou nutraceutiques.

Un produit alimentaire au sens de la présente invention peut être considéré comme toute substance assurant la nutrition d'un être vivant, et particulièrement d'un être humain. Le produit alimentaire peut donc aussi bien se présenter sous une forme solide que sous une forme liquide. A titre d'exemple, le produit alimentaire peut être un plat préparé, une confiture, une boisson fermentée telle que le vin ou la bière, une boisson énergisante ou encore une boisson protéinée pour le sport.

Un produit pharmaceutique au sens de la présente invention désigne toutes substances pharmaceutiques destinées à être ingérées par un être humain, comme par exemple un médicament.

D'une manière tout à fait surprenante, les inventeurs ont constaté qu'un extrait de levure présentant de 25 % à 55 % en poids de 5'-ribonucléotides dont une quantité particulière de 5'-AMP et de 5'-GMP selon un ratio 5'-AMP/5'-GMP de 0,85 à 1,25, permettait lorsqu'il était utilisé dans la préparation de produits, de masquer les saveurs amère et acide et les notes aromatiques indésirables d'édulcorants, de protéines et métalliques. Cet effet est notamment obtenu sans qu'il soit nécessaire de convertir le 5'-AMP en 5'-IMP comme cela était communément réalisé dans l'art antérieur lors de l'utilisation d'extraits de levure riches en 5'-ribonucléotides en tant qu'agent masquant de note d'édulcorant.

D'une manière générale, les extraits de levure sont des produits connus. Par extrait de levure, on comprend selon l'invention, la fraction soluble obtenue après hydrolyse enzymatique de cellules de levure. Egalement selon l'invention, l'extrait de levure est de préférence la fraction soluble obtenue après autolyse desdites cellules de levure, c'est-à-dire après hydrolyse enzymatique effectuée uniquement par les enzymes endogènes de la levure. L'hydrolyse des cellules de levure peut également être réalisée par l'intermédiaire d'enzymes exogènes, c'est-à-dire en ajoutant des enzymes supplémentaires, comme notamment des protéases.

De préférence, l'extrait de levure est séparé de la partie insoluble des cellules de levure. L'extrait de levure ainsi séparé de la partie insoluble offre l'avantage d'une meilleure conservation sans apparition de notes aromatiques dues à l'oxydation des lipides membranaires de la partie insoluble.

L'extrait de levure selon l'invention peut être obtenu à partir de n'importe quelle levure. De préférence, la souche de levure utilisée pour la préparation de l'extrait selon l'invention appartient au genre *Saccharomyces, Kluyveromyces* ou *Candida* (également connue sous les noms *Pichia* ou *Lindnera*). De préférence, la souche de levure utilisée pour la préparation de l'extrait appartient au genre *Saccharomyces* et tout particulièrement à l'espèce *Saccharomyces cerevisiae.*

L'extrait de levure riche en 5'-ribonucléotides selon l'invention peut être obtenu par les procédés connus de l'homme du métier comme ceux décrits par exemple dans l'ouvrage de référence « Yeast Technology » de G.Reed et T.W. Nagodawithana, 2ème édition (Van Nostrand Reinhold, ISBN 0-442-31892-8) pages 382 à 385. Il est notamment connu de préparer des extraits de levure riches en 5'-nucléotides par hydrolyse enzymatique de levures en présence de 5'-phosphodiestérase avec désactivation des phosphatases et nucléases endogènes de la levure. On obtient ainsi des extraits de levure contenant les 5'-ribonucléotides suivants : 5'-GMP, 5'-UMP, 5'-CMP et 5'-AMP.

Parmi les procédés adaptés connus pour la fabrication de dérivés de levure riche en 5'-ribonucléotides, on peut également citer les procédés suivants : Le brevet US-A-4 810 509 décrit un procédé de production d'extrait de levure riche en 5'-nucléotides comprenant (1) une étape de chauffage d'une suspension de levure entre 55°C et 70°C, (2) une étape d'autolyse des cellules de levure à pH 8 à 10, (3) un ajustement du pH de la suspension de levures autolysées entre 5 et 7, (4) une étape de chauffage de cette suspension à 90°C ou plus , (5) l'élimination du matériel insoluble de cette suspension chauffée et (6) la récupération de l'extrait de levure contenant des 5'-nucléotides. Le procédé selon EP-A-0299078 consiste à chauffer une suspension de levure contenant une grande quantité de ARN entre 80°C et 120 °C (destruction des ribonucléases), puis à extraire les ARN avec un traitement alcalin et à les découper en 5'-nucléotides par l'action d'une 5'-phosphodiestérase.

Le procédé selon WO02/067959 consiste à préparer un dérivé de levure par une autolyse réalisée à une température supérieure à 35°C, par exemple comprise entre 35- 70°C, de préférence entre 50-60°C. Les levures sont préférentiellement hydrolysées pendant ou après l'autolyse avec une ou plusieurs protéases. Facultativement, le produit peut être centrifugé et une étape supplémentaire d'ultrafiltration du surnageant peut également être réalisée.

L'homme du métier peut donc adapter les procédés connus de manière à obtenir un extrait de levure comprenant de 25 % à 55 % en poids de 5'-ribonucléotides dont 5 % à 20 % en poids de 5'-AMP et 5 % à 20 % en poids de 5'-GMP selon un ratio 5'-AMP/5'GMP compris entre 0,85 et 1,25.

L'homme du métier sait également qu'un extrait de levure comprenant de 25 % à 55 % en poids de ribonucléotides dont 5 % à 20 % en poids de 5'-AMP et 5 % à 20 % en poids de 5'-GMP peut être obtenu en ajoutant des 5'-ribonucléotides isolés ou synthétisés chimiquement à un extrait de levure comprenant moins de 25 % en poids de 5'-ribonucléotides. Ces extraits de levure font également partie de la présente invention. Ceux obtenus par addition de 5'-ribonucléotides synthétisés chimiquement ne sont pas nécessairement préférés car les produits préparés à partir de tels extraits de levure devront être étiquetés comme contenant un additif chimique.

L'extrait de levure selon l'invention est donc naturellement riche en 5'-ribonucléotides et/ou peut être enrichi par l'addition d'une quantité de 5'-ribonucléotides, lesdits 5'-ribonucléotides étant préférentiellement obtenus à partir de levure.

Dans le contexte de la présente invention, le terme « 5'-ribonucléotides » désigne les 5'-nucléotides en tant que tels ainsi que leur hydrates et autres formes physiologiquement acceptables telles que la forme disodique heptahydratée. En particulier, les 5'-ribonucléotides selon l'invention sont la guanosine 5'-monophosphate (5'-GMP), l'adénosine 5'-monophosphate (5'-AMP), l'uridine 5'-monophosphate (5'-UMP) ou la cytidine 5'-monophosphate (5'-CMP), et l'ionosine 5'-monophosphate (5'-IMP). L'ionosine 5'-monophosphate (5'-IMP) peut notamment être obtenue par conversion du 5'-AMP par l'enzyme 5'-adenylate déaminase (AMP déaminase).

Selon un mode de réalisation particulièrement préféré, les 5'-ribonucléotides ne désignent pas l'ionosine 5'-monophosphate (5'-IMP). Selon ce mode de réalisation, l'extrait de levure selon l'invention est donc exempt de 5'-IMP.

On entend par « extrait de levure riche en 5'-ribonucléotides » un extrait de levure qui comprend de 25 % à 55 % en poids de 5'-ribonucléotides par rapport au poids de matières sèches de l'extrait de levure et calculés en tenant compte de la forme disodique heptahydratée. De préférence, l'extrait de levure comprend de 30 % à 55 % en poids de 5'-ribonucléotides par rapport au poids de matières sèches de l'extrait de levure et tout particulièrement de 35 % à 55 % en poids de 5'-ribonucléotides par rapport au poids de matières sèches de l'extrait de levure.

Parmi les 25 % à 55 % en poids de 5'-ribonucléotides, 5 % à 20 % en poids sont des 5'-AMP et 5 % à 20 % en poids sont des 5'-GMP. De préférence, parmi les 25 % à 55 % en poids de 5'-ribonucléotides, 8 % à 16 % en poids sont des 5'-AMP et 8 % à 16 % en poids sont des 5'-GMP, et de préférence encore, 9 % à 14 % en poids sont des 5'-AMP et 9 % à 14 % en poids sont des 5'-GMP.

D'une manière particulièrement avantageuse, l'extrait de levure comprend de 40 % à 55 % en poids de 5'-ribonucléotides, dont environ 10 % en poids sont des 5'-AMP et environ 10 % en poids sont des 5'-GMP. L'extrait de levure selon l'invention comprend également moins de 15 % en poids de 5'-CMP, de préférence, moins de 12 % en poids de 5'-CMP, et tout particulièrement, moins de 9 % en poids de 5'-CMP. Les pourcentages en poids étant également exprimés par rapport au poids de matières sèches de l'extrait de levure et sont calculés en tenant compte de la forme disodique heptahydratée des 5'-ribonucléotides.

Dans l'extrait de levure selon l'invention le ratio 5'-AMP/5'-GMP est de 0,85 à 1,25. De préférence, le ratio 5'-AMP/5'-GMP est de 0,90 à 1,15, de préférence de 0,95 à 1,10, de préférence encore de 0,98 à 1,05 et tout particulièrement, le ratio 5'-AMP/5'-GMP est environ égal à 1,00.

L'extrait de levure selon l'invention présente également un taux d'acides aminés libres de 0 % à 20 %, de préférence de 1 % à 10 %, et un taux d'acides aminés totaux de 25 % à 55 %, et de préférence de 35 % à 45 %.

L'extrait de levure selon l'invention peut se présenter sous les formes connues de l'homme du métier. D'une manière préférentielle, l'extrait de levure se présente sous la forme d'un extrait sec.

Selon un mode de réalisation particulier, l'extrait de levure selon l'invention peut se présenter sous une forme de poudre ou sous une forme liquide. De préférence, l'extrait de levure est sous une forme de poudre.

Selon un autre mode de réalisation particulier, l'extrait de levure selon l'invention peut être dilué dans un support ou un excipient physiologiquement acceptable. Un support ou excipient physiologiquement acceptable est un support ou excipient aromatiquement neutre et approprié à l'administration chez l'homme. Des exemples de supports ou d'excipients physiologiquement acceptables sont notamment les maltodextrines, le triacétine, le propylène glycol, la glycérine végétale, le glycérol, les fibres solubles, des dérivés de levure tels que les extraits de levures, les écorces et les autolysats, ou encore des matières grasses telles que l'huile de palme.

Un autre objet de l'invention concerne l'utilisation d'un extrait de levure tel que défini précédemment pour le masquage des saveurs amère et acide et des notes indésirables d'édulcorants, de protéines et métalliques dans un produit.

Au sens de la présente invention, le produit peut aussi bien être un produit alimentaire, pharmaceutique, nutraceutique ou encore une préparation aromatique.

L'extrait de levure utilisé selon l'invention comprend de 25 % à 55 % en poids de 5'-ribonucléotides par rapport au poids de matières sèches de l'extrait de levure. De préférence, l'extrait de levure comprend de 30 % à 55 % en poids de 5'-ribonucléotides et tout particulièrement de 35 % à 55 % en poids de 5'-ribonucléotides par rapport au poids de matières sèches de l'extrait de levure.

Parmi les 25 % à 55 % en poids de 5'-ribonucléotides, 5 % à 20 % en poids sont des 5'-AMP et 5 % à 20 % en poids sont des 5'-GMP. De préférence, parmi les 25 % à 55 % en poids de 5'-ribonucléotides, 8 % à 16 % en poids sont des 5'-AMP et 8 % à 16 % en poids sont des 5'-GMP, et de préférence encore, 9 % à 14 % en poids sont des 5'-AMP et 9 % à 14 % en poids sont des 5'-GMP.

D'une manière particulièrement avantageuse, l'extrait de levure comprend de 40 % à 55% en poids de 5'-ribonucléotides, dont environ 10 % en poids sont des 5'-AMP et environ 10 % en poids sont des 5'-GMP.

Dans l'extrait de levure selon l'invention le ratio 5'-AMP/5'-GMP est de 0,85 à 1,25. De préférence, le ratio 5'-AMP/5'-GMP est de 0,90 à 1,15, de préférence de 0,95 à 1,10, de préférence encore de 0,98 à 1,05 et tout particulièrement, le ratio 5'-AMP/5'-GMP est environ égal à 1,00.

L'extrait de levure utilisé selon l'invention comprend également moins de 15 % en poids de 5'-CMP, de préférence, moins de 12 % en poids de 5'-CMP, et tout particulièrement, moins de 9 % en poids de 5'-CMP. Les pourcentages en poids étant également exprimés par rapport au poids de matières sèches de l'extrait de levure.

Selon un mode de réalisation particulièrement préféré, l'extrait de levure selon l'invention est exempt de 5'-IMP.

L'extrait de levure selon l'invention présente également un taux d'acides aminés libres de 0 % à 20 %, de préférence de 1 % à 10 %, et un taux d'acides aminés totaux de 25 % à 55 %, et de préférence de 35 % à 45 %.

Les quantités d'extrait de levure présentent au sein du produit alimentaire peuvent être de 10 ppm à 1000 ppm. De préférence, les quantités d'extrait de levure sont de 10 ppm à 500 ppm, de préférence encore de 50 ppm à 200 ppm, et tout particulièrement d'environ 100 ppm. D'une manière surprenante, lorsque la quantité d'extrait de levure dépasse les 1000 ppm, l'extrait de levure ne permet plus d'obtenir les différents masquages, et au contraire, la saveur et les notes indésirables s'en trouvent augmentées.

Selon un mode de réalisation particulier, l'extrait de levure utilisé selon l'invention peut se présenter sous une forme de poudre ou sous une forme liquide. De préférence, l'extrait de levure est sous une forme de poudre.

Selon un autre mode de réalisation particulier, l'extrait de levure utilisé selon l'invention peut être dilué dans un support ou un excipient physiologiquement acceptable. Un support ou excipient physiologiquement acceptable est un support ou excipient aromatiquement neutre et approprié à l'administration chez l'homme. Des exemples de supports ou d'excipients physiologiquement acceptables sont notamment les maltodextrines, le triacétine, le propylène glycol, la glycérine végétale, le glycérol, les fibres solubles, des dérivés de levure tels que les extraits de levures, les écorces et les autolysats, ou encore des matières grasses telles que l'huile de palme.

Spécifiquement, l'utilisation d'un extrait de levure selon l'invention permet le masquage des saveurs amère et acide et de notes indésirables d'édulcorants, de protéines et métalliques dans un produit alimentaire ou un produit pharmaceutique. Ainsi, l'extrait de levure permet de faire plusieurs masquages, à l'inverse de ce qui était jusqu'à présent connu.

L'utilisation selon l'invention est également particulièrement avantageuse en ce que l'extrait de levure, lorsqu'il est incorporé aux dosages préconisés au sein du produit alimentaire ou pharmaceutique n'engendre pas de note de bouillon, de note aromatique ou de saveur umami généralement caractéristiques des extraits de levure.

Comme mentionné précédemment, le produit alimentaire peut être considéré comme toute substance assurant la nutrition d'un être vivant, et particulièrement d'un être humain, et peut se présenter aussi bien sous une forme solide que sous une forme liquide. A titre d'exemple, le produit alimentaire peut être un plat préparé, une confiture, une boisson fermentée telle que le vin ou la bière, une boisson énergisante ou encore une boisson protéinée pour le sport.

De même ; le produit pharmaceutique désigne toutes substances pharmaceutiques destinées à être ingérées par un être humain, comme par exemple un médicament.

La notion de masquage dans un produit fait référence au fait que la présence des extraits de levure selon l'invention au sein dudit produit permet de diminuer, voire de supprimer complètement des saveurs indésirables et des notes indésirables. Le masquage selon l'invention s'entend donc comme l'absence desdites saveurs et desdites notes, ou tout au moins leur diminution. L'absence ou la diminution des saveurs et des notes indésirables est mise en évidence par la perception sensorielle humaine car à ce jour, les autres méthodes telles par exemple que l'utilisation d'une langue électronique, ne permettent pas une détection fine d'un masquage particulier. En effet, les méthodes actuelles de détection ne sont pas suffisamment représentatives du mécanisme de perception humaine qui dépend d'une multitude de facteurs, dont certains ne sont d'ailleurs pas uniquement physiologiques.

Un autre objet de l'invention concerne un procédé de masquage des saveurs amère et acide et de notes indésirables d'édulcorants, de protéines et métalliques dans un produit.

Le procédé de masquage selon l'invention comprend les étapes suivantes:
- de fourniture d'un extrait de levure tel que défini précédemment,
- d'incorporation dudit extrait de levure dans un produit.

Le procédé selon l'invention permet de masquer les saveurs amère et acide et les notes indésirables d'édulcorants, de protéines et métalliques au sein d'un produit. Le produit peut par exemple aussi bien être un produit alimentaire, pharmaceutique, nutraceutique ou encore une préparation aromatique. A titre d'exemple de produit alimentaire, on peut citer un plat préparé, une confiture, une boisson fermentée telle que le vin ou la bière, une boisson énergisante ou encore une boisson protéinée pour le sport. A titre d'exemple de produit pharmaceutique, on peut citer un médicament.

Selon le procédé de l'invention, l'extrait de levure fourni comprend notamment de 25 % à 55 % en poids de 5'-ribonucléotides, dont 5 % à 20 % en poids d'adénosine 5'-monophosphate (5'-AMP) et 5 % à 20 % en poids de guanosine 5'-monophosphate (5'-GMP) selon un ratio 5'-AMP/5'GMP allant de 0,85 à 1,25.

L'étape d'incorporation est une étape connue de l'homme du métier et consiste notamment à appliquer et/ou mélanger l'extrait de levure au produit. Cette étape d'incorporation peut donc aussi bien être réalisée pendant la fabrication dudit produit qu'après, et peut également être réalisée à chaud ou à froid.

Les quantités d'extrait de levure à incorporer peuvent être de 10 ppm à 1000 ppm. De préférence, les quantités d'extrait de levure à incorporer sont de 10 ppm à 500 ppm, de préférence encore de 50 ppm à 200 ppm, et tout particulièrement d'environ 100 ppm. D'une manière surprenante, lorsque la quantité d'extrait de levure à incorporer dépasse les 1000 ppm, le masquage des saveurs amère et acide n'est plus présent, et à l'inverse, lesdites saveurs sont plus prononcées dans le produit.

Selon un mode de réalisation particulier, l'extrait de levure fourni selon la première étape du procédé de l'invention peut se présenter sous une forme de poudre ou sous une forme liquide. De préférence, l'extrait de levure est sous une forme de poudre.

Selon un autre mode de réalisation particulier, l'extrait de levure fourni selon la première étape du procédé de l'invention peut être dilué dans un support ou un excipient physiologiquement acceptable. Un support ou excipient physiologiquement acceptable est un support ou excipient aromatiquement neutre et approprié à l'administration chez l'homme. Des exemples de supports ou d'excipients physiologiquement acceptables sont notamment les maltodextrines, le triacétine, le propylène glycol, la glycérine végétale, le glycérol, les fibres solubles, des dérivés de levure tels que les extraits de levures, les écorces et les autolysats, ou encore des matières grasses telles que l'huile de palme.

L'invention sera mieux comprise à l'aide des exemples qui suivent, lesquels se veulent purement illustratifs et ne limitent en rien la portée de l'invention.

### EXEMPLES

Une étude organoleptique comparative a été menée par un panel de 7 experts sur des produits préparés ou non à partir d'un extrait de levure selon l'invention.

### Exemple 1 : masquage de note indésirable d'édulcorant

Dans cet exemple, le masquage note indésirable d'édulcorant par un extrait de levure selon l'invention a été mis en évidence dans une confiture et dans un thé glacé.

### Extraits de levure:

Deux extraits de levure ont été préparés. Le premier (EXL1) est un extrait de levure selon l'invention, riche en 5'-ribonucléotides comprenant une quantité de 5'-AMP et 5'-GMP comprise entre 10 % et 15 % en poids et selon un ratio 5'-AMP/5'-GMP de 0,98. EXL1 est préparé à partir d'une souche de levure de l'espèce *Saccharomyces cerevisiae.* Les souches de levure sont d'abord soumises à un choc thermique à une température supérieure à 75°C avant qu'une enzyme, la phosphodiestérase, soit ajoutée à un pH compris entre 4,5 et 7,5. Les souches sont ensuite soumises à une hydrolyse pendant 8 à 24h à une température comprise entre 40 et 70°C, puis une étape de séparation est réalisée. Le surnageant est alors récupéré, concentré et séché afin d'obtenir l'extrait EXL1 selon l'invention.

Le second extrait (EXL2) est un extrait de levure commercial vendu par la Demanderesse sous la référence Springer^{®}2020. Cet extrait est différent des extraits de levure selon l'invention en ce qu'il ne contient notamment pas de 5'-AMP, il est donc utilisé en tant que comparatif.

Les quantités des différents 5'-ribonucléotides au sein des extraits de levure sont reprises dans le tableau 1 ci-dessous :

**TABLEAU 1**

| **EXL 1** | | **EXL 2 (comparatif)** | |
|---|---|---|---|
| - 50,9 % en poids de 5'-ribonucléotides, dont : | | - 50,9 % en poids de 5'-ribonucléotides, dont : | |
| | - 10 % en poids de 5'-AMP | | - 10,1 % en poids de 5'-IMP |
| | - 10,1 % en poids de 5'-GMP | | - 10,2 % en poids de 5'-GMP |

### Produits alimentaires utilisés pour la mise en évidence du masquage:

- Produit 1 (contrôle): Confiture commerciale Gerblé^{®} à l'abricot comprenant 0,03-0,04 % de sucralose en tant qu'édulcorant. Les pourcentages étant exprimés par rapport au poids total de la confiture.
- Produit 2 (comparatif): Confiture commerciale Gerblé^{®} à l'abricot mélangée uniformément à 100 ppm d'EXL2
- Produit 3 : Confiture commercial Gerblé^{®} à l'abricot mélangée uniformément à 100 ppm d'EXL1.
- Produit 4 : Confiture commerciale Gerblé^{®} à l'abricot mélangée uniformément à 2000 ppm d'EXL1.
- Produit 5 (contrôle): Thé glacé à base de stévia comprenant 0,035 % de stéviosides en tant qu'édulcorant. Les pourcentages étant exprimés par rapport au poids total de boisson.
- Produit 6 (comparatif): Thé glacé à base de stévia mélangé uniformément à 100 ppm d'EXL2.
- Produit 7 : Thé glacé à base de stévia mélangé uniformément à 100 ppm d'EXL1.

Les compositions des produits 5, 6 et 7 sont reprises dans le tableau 2 ci-dessous :

**TABLEAU 2**

| **Thé glacé** | **Produit 5** | **Produit 6** | **Produit 7** |
|---|---|---|---|
| Eau | 99,78 | 99,77 | 99,77 |
| Acide citrique | 0,15 | 0,15 | 0,15 |
| Extrait de thé vert | 0,02 | 0,02 | 0,02 |
| Arôme MANE Peach M57337 | 0,01 | 0,01 | 0,01 |
| Stévia 97% Reb A | 0,035 | 0,035 | 0,035 |
| EXL 1 | | | 0,0100 |
| EXL 2 | | 0,0100 | |
| TOTAL | 100,00 | 100,00 | 100,00 |

Les produits ont ensuite été soumis à un panel d'experts pour une analyse organoleptique comparative. Chaque expert a goûté le produit contrôle pour une comparaison à l'aveugle avec les produits contenant les extraits de levure. Pour les produits contenant les extraits de levure, les membres du panel devaient également déterminer l'absence ou la présence de notes aromatiques de levure. Les résultats sont les suivants :
Produit 1 : L'arôme abricot est prononcé, présence d'une acidité en bouche et d'une note d'édulcorant.
Produit 2 : L'arôme abricot est toujours aussi prononcé, saveur sucrée moins présente, l'acidité en bouche est plus prononcée que le produit 1, la note d'édulcorant est moins prononcée mais toujours présente. Les experts du panel identifient des notes aromatiques de levure.
Produit 3 : L'arôme abricot est plus prononcé, tout comme la saveur sucrée, et pas de présence de note d'édulcorant. Les experts du panel relèvent l'absence de notes aromatiques de levure.
Produit 4 : l'arôme abricot est toujours aussi prononcé, présence de note d'édulcorant. Les experts du panel identifient également des notes aromatiques de levure.
Produit 5 : Saveur sucrée prononcée, présence d'arômes fruités, présence de note d'édulcorant qui persiste également après la déglutition.
Produit 6 : Saveur sucrée moins prononcée, la note d'édulcorant est moins prononcée mais toujours présente. Les experts du panel identifient des notes aromatiques de levure dans le thé glacé.
Produit 7 : Saveur sucrée moins prononcée et présence d'une légère acidité. Absence de note d'édulcorant. Les experts du panel n'identifient pas de note aromatique de levure dans le thé glacé.

Par l'absence de détection de note d'édulcorant par le panel d'experts, cet exemple démontre bien la capacité de masquage de l'extrait de levure selon l'invention, ledit extrait étant également avantageux en ce qu'aucune note aromatique de levure n'est présente dans le produit alimentaire. De même le produit 4 comparatif démontre l'importance du dosage de l'extrait de levure selon l'invention pour obtenir le masquage de note d'édulcorant. En effet, un dosage trop important ne permet pas d'obtenir ledit masquage.

### Exemple 2 : masquage de la saveur amère

Dans cet exemple, le masquage d'arrière-goût d'amertume par un extrait de levure selon l'invention a été mis en évidence dans un coulis au caramel.

### Extraits de levure:

Deux extraits de levure ont été préparés et utilisés. Il s'agit des mêmes extraits (EXL1 et EXL2) que ceux de l'exemple 1 précédent.

### Produits alimentaires utilisés pour la mise en évidence du masquage:

- Produit 8 (contrôle): Coulis caramel commercial Vahiné^{®}
- Produit 9 (comparatif): Coulis caramel commercial Vahiné^{®} mélangé uniformément à 100 ppm d'EXL2
- Produit 10 : Coulis caramel commercial Vahiné^{®} mélangé uniformément à 100 ppm d'EXL1

Selon le même protocole que pour l'exemple 1, chaque produit a été soumis à un panel d'experts pour l'analyse organoleptique comparative et les résultats sont les suivants :
Produit 8 : Arôme de caramel prononcé, présence d'un arôme de brulé, saveur sucrée très prononcée et présence de saveur amère.
Produit 9 : Arôme de caramel toujours prononcé par rapport au produit 8, saveur sucrée toujours aussi prononcée, présence similaire d'une saveur amère. De plus, les experts du panel identifient des notes aromatiques de levure.
Produit 10 : Arôme de caramel toujours prononcé et saveur sucrée plus prononcée en bouche que le produit 8. Par contre, les experts relèvent l'absence de saveur amère et l'absence de notes aromatiques de levure.

L'étude par le panel d'experts démontre ainsi que l'extrait de levure selon l'invention permet d'obtenir un produit ayant une saveur sucrée plus prononcée en bouche que le produit original tout en réalisant un masquage de la saveur amère et sans pour autant engendrer de notes aromatiques de levure dans le produit alimentaire.

### Exemple 3 : masquage de notes indésirables de protéines

### a) Solution de protéines de pois

Dans cet exemple, le masquage de notes de protéines par un extrait de levure selon l'invention a été mis en évidence dans une eau protéinée.

### Extraits de levure:

Deux extraits de levure ont été préparés. Il s'agit des mêmes extraits (EXL1 et EXL2) que ceux de l'exemple 1 précédent.

### Produits alimentaires utilisés pour la mise en évidence du masquage:

- Produit 11 (contrôle): Solution à 3% de protéines de pois Peatex^{®} dans l'eau.
- Produit 12 (comparatif): Solution à 3% de protéines de pois Peatex^{®} dans l'eau mélangée uniformément à 50 ppm d'EXL 2
- Produit 13 : Solution à 3% de protéines de pois Peatex^{®} dans l'eau mélangée uniformément à 50 ppm d'EXL 1.

Selon le même protocole que pour les exemples précédents, chaque produit a été soumis au panel d'experts pour l'analyse organoleptique comparative et les résultats sont les suivants :
Produit 11 : Solution amère, présence de note indésirable de protéines de pois et de note indésirable métallique.
Produit 12 : Solution toujours autant amère, note métallique aussi prononcée et très légère diminution des notes indésirables de protéines de pois. Les experts du panel identifient des notes aromatiques de levure.
Produit 13 : Solution moins amère que le contrôle, note indésirable métallique moins prononcée et diminution importante de note indésirable de protéines de pois. Les experts du panel relèvent l'absence de note aromatique de levure.

Les résultats de l'étude organoleptique démontrent ainsi les propriétés de masquage de note de protéines. En effet, l'ajout des extraits de levure selon l'invention (EXL1) entraine une nette diminution de note indésirable de protéines mais également d'une manière avantageusement une diminution de la saveur amère, et sans toutefois engendrer de note aromatique de levure.

### b) Produit vegan de type « poulet sauce tomate »

Le masquage de note de protéines par un extrait de levure selon l'invention a également été mis en évidence dans un produit vegan de type « poulet sauce tomate ».

### Extraits de levure:

Deux extraits de levure ont été préparés. Il s'agit des mêmes extraits (EXL1 et EXL2) que ceux de l'exemple 1 précédent.

### Produits alimentaires utilisés pour la mise en évidence du masquage:

- Produit 14 (contrôle): plat préparé en conserve à base de protéines de blé texturées de marque Trutex^{®} 1501 (MGP Ingredients).
- Produit 15 : plat préparé en conserve à base de protéines de blé texturées de marque Trutex^{®} 1501 mélangé uniformément à 100 ppm d'EXL 1.

Les compositions des produits 14 et 15 sont reprises dans le tableau 3 ci-dessous :

**TABLEAU 3**

| **Plat préparé en conserve à base de protéines de blé texturées** | **Produit 14** | **Produit 15** |
|---|---|---|
| Eau | 85,85 | 85,84 |
| Trutex 1501 (protéines de blé hydrolysées) | 7,00 | 7,00 |
| concentré de tomate | 2,00 | 2,00 |
| sucre | 1,00 | 1,00 |
| maltodextrine | 1,00 | 1,00 |
| huile de tournesol | 1,00 | 1,00 |
| Amidon de maïs | 0,80 | 0,80 |
| sel | 0,60 | 0,60 |
| Poudre d'oignons grillés | 0,50 | 0,50 |
| Gomme xanthane | 0,10 | 0,10 |
| Poudre de laurier | 0,10 | 0,10 |
| herbes de Provence | 0,05 | 0,05 |
| EXL1 | | 0,01 |
| TOTAL | 100,00 | 100,00 |

Les compositions ont été mises en conserve et appertisées 12 minutes à 121°C.

Selon le même protocole que pour les exemples précédents, chaque produit a été soumis au panel d'experts pour l'analyse organoleptique comparative et les résultats sont les suivants :
Produit 14 : présence d'une note indésirable de protéine végétale et de note indésirable métallique.
Produit 15 : diminution importante de l'arrière-note de protéine végétale. Les experts du panel relèvent l'absence de note aromatique de levure.

Cette étude organoleptique démontre ainsi de nouveau les propriétés de masquage de l'extrait selon l'invention. En effet, l'ajout des extraits de levure selon l'invention (EXL1) entraine une nette diminution de la note aromatique de protéine végétale et de la note indésirable métallique sans toutefois engendrer de note aromatique de levure.

### Exemple 4: masquage de note indésirable métallique

Dans cet exemple, le masquage de note métallique par un extrait de levure selon l'invention a été mis en évidence dans une soupe riche en chlorure de potassium (KCl).

### Extraits de levure:

Deux extraits de levure ont été préparés. Il s'agit des mêmes extraits (EXL1 et EXL2) que ceux de l'exemple 1 précédent.

### Produits alimentaires utilisés pour la mise en évidence du masquage:

- Produit 16 (contrôle): Soupe commerciale à la tomate Campbell^{®} contenant 0,25 % de KCI.
- Produit 17 (comparatif): Soupe commerciale à la tomate Campbell^{®} mélangée uniformément à 100 ppm d'EXL 2.
- Produit 18 : Soupe commerciale à la tomate Campbell^{®} mélangée uniformément à 100 ppm d'EXL 1.

Selon le même protocole que les exemples précédents, chaque produit a été soumis aux experts du panel pour l'analyse organoleptique comparative et les résultats sont les suivants :
Produit 16 : Arôme tomate prononcé, présence d'une saveur sucrée, présence d'une légère saveur amère, et présence très prononcée de note indésirable métallique due notamment au chlorure de potassium.
Produit 17: l'arôme tomate est autant prononcé que le produit 14, la saveur sucrée est légèrement plus prononcée, la saveur amère et la note indésirable métallique sont toujours prononcées. Les experts du panel identifient des notes aromatiques de levure.
Produit 18 : La saveur amère est moins prononcé. La note indésirable métallique est fortement diminuée comparativement au produit 14. Les experts du panel relèvent l'absence de note aromatique de levure.

Les résultats montrent ainsi l'effet bénéfique de l'extrait de levure selon l'invention sur le masquage de note métallique et sans toutefois induire de note aromatique de levure dans le produit obtenu.

### Exemple 5: masquage de la saveur acide

Dans cet exemple, le masquage de la note « acide » par un extrait de levure selon l'invention a été mis en évidence dans une confiture de marque Bonne Maman^{®}

### Extrait de levure:

Deux extraits de levure ont été préparés. Il s'agit des mêmes extraits (EXL1 et EXL2) que ceux de l'exemple 1 précédent.

### Produits alimentaires utilisés pour la mise en évidence du masquage:

- Produit 19 (contrôle): Confiture commerciale de marque Bonne maman^{®} à la fraise.
- Produit 20 (comparatif): Confiture commerciale de marque Bonne maman^{®} à la fraise mélangée uniformément à 100 ppm d'EXL 2.
- Produit 21 : Confiture commerciale de marque Bonne maman^{®} à la fraise mélangée uniformément à 100 ppm d'EXL 1.

Les produits ont ensuite été soumis à un panel d'experts pour une analyse organoleptique comparative. Chaque expert a goûté le produit contrôle pour une comparaison avec le produit contenant les extraits de levure. Les membres du panel devaient également déterminer l'absence ou la présence de notes aromatique de levure. Les résultats sont les suivants :
Produit 19 : l'arôme fraise est prononcé, présence d'une saveur sucrée lors de la mise en bouche et surtout présence d'un pic de la saveur acide dans le produit.
Produit 20 : La saveur sucrée est plus prononcée, l'arôme fraise est moins présent et la saveur acide est toujours aussi présente. Les experts du panel identifient la présence de légères notes aromatiques de levure en fin de bouche.
Produit 21 : La saveur sucrée est légèrement plus prononcée mais surtout plus prolongée dans le temps, alors que la saveur acide est absente du produit. Les experts n'identifient pas de note aromatique de levure dans le produit.

Les résultats montrent ainsi l'effet bénéfique de l'extrait de levure selon l'invention sur le masquage de la saveur acide et sans l'induction de notes aromatiques de levure dans le produit alimentaire.

Les exemples 1 à 5 ont été reproduits en remplaçant l'extrait de levure EXL1 soit par l'extrait de levure EXL3, soit par l'extrait de levure EXL4, lesdits extraits EXL3 et EXL4 étant également des extraits selon l'invention et dont les compositions sont reprises dans le tableau 4 ci-après :

**TABLEAU 4**

| **EXL 3** | | **EXL 4** | |
|---|---|---|---|
| - 44,8 % en poids de 5'-ribonucléotides, dont : | | - 39,91 % en poids de 5'-ribonucléotides, dont : | |
| | - 11,9 % en poids de 5'-AMP | | - 10,13 % en poids de 5'-AMP |
| | - 11,8 % en poids de 5'-GMP | | - 11,57 % en poids de 5'-GMP |

Les résultats des études organoleptiques menées par le même panel d'experts sont les mêmes que ceux obtenus avec l'extrait de levure EXL1, et démontrent ainsi de nouveau les effets bénéfiques des extraits de levure selon l'invention sur le masquage des saveurs amère et acide et des notes indésirables d'édulcorants, de protéines et métalliques.

### Exemple 6: Effet sur le masquage d'un extrait de levure selon l'invention mélangé à un support aromatiquement neutre :

Le support aromatiquement neutre utilisé pour cet exemple est la maltodextrine Glucidex^{®} 12, vendue par la Société Roquette.

L'extrait de levure EXL 1 décrit précédemment a été mélangé à cette maltodextrine afin d'obtenir un mélange final présentant une quantité de 5'-AMP et 5'-GMP égale à 10 % en poids et un ratio 5'-AMP/5'-GMP de 0,98.

Le mélange de maltodextrine et d'extrait EXL 1 ainsi obtenu a ensuite été ajouté dans les produits listés ci-dessous et décrits aux exemples précédents, à savoir :
- Produit 5 : thé glacé à base de stévia comprenant 0,035 % de stéviosides en tant qu'édulcorant. Les pourcentages étant exprimés par rapport au poids total de boisson,
- Produit 11 : solution à 3% de protéines de pois Peatex^{®} dans l'eau,
- Produit 16 : soupe commerciale à la tomate Campbell^{®} contenant 0,25 % de KCl,
- Produit 19 : confiture commerciale de marque Bonne maman^{®} à la fraise.

Les quantités du mélange de maltodexrines et de l'extrait EXL 1 ajoutées sont respectivement de 100 ppm pour les produits 5, 16 et 19, et de 50 ppm pour le produit 11.

Les produits obtenus ont ensuite été soumis au panel d'experts pour une analyse organoleptique comparative.

Les effets sur le masquage du mélange de maltodextrine et d'extrait EXL 1 dans les produits 5, 11, 16 et 19 ont été comparés avec ceux obtenus dans les mêmes produits mais en présence de l'extrait de levure EXL 1 seul (produits 7, 13, 18 et 21).

Les résultats montrent que l'ajout de maltodextrine à l'extrait de levure EXL1 n'altère pas les propriétés de masquage vis-à-vis de la saveur acide et des notes indésirables d'édulcorant, de protéines et métalliques.

## Revendications

1. Extrait de levure riche en 5'-ribonucléotides comprenant de 25 % à 55 % en poids de 5'-ribonucléotides **caractérisé en ce qu'**il comprend 5 % à 20 % en poids de 5'-AMP et 5 % à 20 % en poids de 5'-GMP, selon un ratio 5'-AMP/5'GMP compris entre 0,85 et 1,25, les pourcentage en poids étant exprimés par rapport au poids de matières sèches de l'extrait de levure.

2. Extrait de levure selon la revendication 1, **caractérisé en ce qu'**il est obtenu à partir d'une souche de levure de l'espèce *Saccharomyces cerevisiae.*

3. Extrait de levure selon la revendication 1 ou 2, **caractérisé en ce qu'**il est exempt de 5'-IMP.

4. Extrait de levure selon l'une des revendications 1 à 3, **caractérisé en ce qu'**il comprend de 35 % à 55 % en poids de 5'-ribonucléotides.

5. Extrait de levure selon l'une des revendications 1 à 4, **caractérisé en ce qu'**il comprend de 8 % à 16 % en poids de 5'-AMP et 8 % à 16 % en poids de 5'-GMP, de préférence de 9 % à 14 % en poids de 5'-AMP et 9 % à 14 % en poids de 5'-GMP.

6. Extrait de levure selon l'une des revendications 1 à 5, **caractérisé en ce que** le ratio 5'-AMP/5'-GMP est compris de 0,85 à 1,25, de préférence de 0,90 à 1,15, de préférence de 0,95 à 1,10, de préférence encore de 0,98 à 1,05, et tout particulièrement environ égal à 1.

7. Extrait de levure selon l'une des revendications 1 à 6, **caractérisé en ce qu'**il comprend un taux d'acides aminés libres de 0 % à 20% et un taux d'acides aminés totaux de 25 % à 55 %.

8. Utilisation d'un extrait de levure tel que défini selon l'une des revendications 1 à 7, pour le masquage des saveurs amère et acide et de notes d'édulcorants, de protéines et métalliques dans un produit.

9. Utilisation selon la revendication 8, **caractérisé en ce que** le produit est un produit alimentaire, pharmaceutique, nutraceutiques, ou une préparation aromatique.

10. Utilisation selon l'une des revendications 8 ou 9, **caractérisée en ce que** l'extrait de levure est présent dans le produit alimentaire selon une quantité comprise entre 10 ppm et 1000 ppm, de préférence entre 50 ppm et 200 ppm.

11. Procédé de masquage des saveurs amère et acide et de notes indésirables d'édulcorants, de protéines et métalliques dans un produit comprenant l'incorporation de 10 à 1000 ppm d'un extrait de levure tel que défini selon l'une quelconque des revendications 1 à 7 dans ledit produit.

12. Procédé de masquage selon la revendication 11, **caractérisé en ce que** l'incorporation de l'extrait de levure dans le produit est réalisée pendant ou après la fabrication dudit produit.

## Patentansprüche

1. Hefeextrakt, reich an 5'-Ribonukleotiden, umfassend 25 bis 55 Gewicht-% von 5'-Ribonukleotiden, **dadurch gekennzeichnet, dass** er 5 bis 20 Gewicht-% von 5'-AMP und 5 bis 20 Gewicht-% von 5'-GMP umfasst, gemäß einem Verhältnis 5'-AMP/5'-GMP, welches zwischen 0,85 und 1,25 beträgt, wobei die Gewicht-Prozentzahlen bezüglich des Gewichts der Trockenmasse des Hefeextrakts ausgedrückt sind.

2. Hefeextrakt nach Anspruch 1, **dadurch gekennzeichnet, dass** er ausgehend von einem Hefestamm der Spezies Saccharomyces cerevisiae erhalten ist.

3. Hefeextrakt nach Anspruch 1 oder 2, **dadurch gekennzeichnet, dass** er frei von 5'-IMP ist.

4. Hefeextrakt nach einem der Ansprüche 1 bis 3, **dadurch gekennzeichnet, dass** er 35 bis 55 Gewicht-% von 5'-Ribonukleotiden umfasst.

5. Hefeextrakt nach einem der Ansprüche 1 bis 4, **dadurch gekennzeichnet, dass** er 8 bis 16 Gewicht-% von 5'-AMP und 8 bis 16 Gewicht-% von 5'-GMP umfasst, vorzugsweise 9 bis 14 Gewicht-% von 5'-AMP und 9 bis 14 Gewicht-% von 5'-GMP.

6. Hefeextrakt nach einem der Ansprüche 1 bis 5, **dadurch gekennzeichnet, dass** das Verhältnis 5'-AMP/5'-GMP zwischen 0,85 und 1,25 beträgt, vorzugsweise zwischen 0,90 und 1,15, vorzugsweise zwischen 0,95 und 1,10, weiter vorzugsweise zwischen 0,98 und 1,05 und am bevorzugtesten etwa gleich 1 ist.

7. Hefeextrakt nach einem der Ansprüche 1 bis 6, **dadurch gekennzeichnet, dass** er einen Gehalt von freien Aminosäuren von 0% bis 20% und einen Gesamtgehalt von Aminosäuren von 25 bis 55% umfasst.

8. Verwendung eines Hefeextrakts, wie in einem der Ansprüche 1 bis 7 definiert, zum Maskieren bitterer und saurer Aromen und von Noten von Farbstoffen, Proteinen und Metallen in einem Produkt.

9. Verwendung nach Anspruch 8, **dadurch gekennzeichnet, dass** das Produkt ein Lebensmittelprodukt, ein Arzneimittel, ein Nutrazeutikum oder eine aromatische Zubereitung ist.

10. Verwendung nach einem der Ansprüche 8 oder 9, **dadurch gekennzeichnet, dass** der Hefeextrakt in dem Lebensmittelprodukt gemäß einer Menge vorliegt, welche zwischen 10 ppm und 1000 ppm beträgt, vorzugsweise zwischen 50 ppm und 200 ppm.

11. Verfahren zum Maskieren bitterer und saurer Aromen und von unerwünschten Noten von Farbstoffen, Proteinen und Metallen in einem Produkt, umfassend ein Aufnehmen von 10 bis 1000 ppm eines Hefeextrakts, wie in einem der Ansprüche 1 bis 7 definiert, in diesem Produkt.

12. Verfahren zum Maskieren nach Anspruch 11, **dadurch gekennzeichnet, dass** das Aufnehmen des Hefeextrakts in das Produkt während oder nach der Herstellung des Produkts durchgeführt wird.

## Claims

1. A 5'-ribonucleotide-rich yeast extract comprising from 25% to 55% by weight of 5'-ribonucleotides, **characterised in that** it comprises from 5% to 20% by weight of 5'-AMP and from 5% to 20% by weight of 5'-GMP, in a 5'-AMP/5'GMP ratio of between 0.85 and 1.25, the weight percentages being expressed relative to the weight of dry matter in the yeast extract.

2. The yeast extract according to claim 1, **characterised in that** it is obtained from a yeast strain of *Saccharomyces cerevisiae* species.

3. The yeast extract according to claim 1 or 2, **characterised in that** it is free of 5'-IMP.

4. The yeast extract according to one of claims 1 to 3, **characterised in that** it comprises from 35% to 55% by weight of 5'-ribonucleotides.

5. The yeast extract according to one of claims 1 to 4, **characterised in that** it comprises from 8% to 16% by weight of 5'-AMP and from 8% to 16% by weight of 5'-GMP, preferably from 9% to 14% by weight of 5'-AMP and from 9% to 14% by weight of 5'-GMP.

6. The yeast extract according to one of claims 1 to 5, **characterised in that** the 5'-AMP/5'-GMP ratio is from 0.85 to 1.25, preferably from 0.90 to 1.15, preferably from 0.95 to 1.10, preferably still from 0.98 to 1.05, and more particularly equal to about 1.

7. The yeast extract according to one of claims 1 to 6, **characterised in that** it comprises a free amino acid level from 0% to 20% and a total amino acid level from 25% to 55%.

8. A use of a yeast extract as defined according to one of claims 1 to 7, for masking bitter and acidic flavours and sweetener, protein and metallic notes in a product.

9. The use according to claim 8, **characterised in that** the product is a food, pharmaceutical, nutraceutical product, or an aromatic preparation.

10. The use according to one of claims 8 or 9, **characterised in that** the yeast extract is present in the food product in an amount of between 10 ppm and 1000 ppm, preferably between 50 ppm and 200 ppm.

11. A method for masking bitter and acidic flavours and undesirable sweetener, protein and metallic notes in a product comprising incorporating from 10 to 1000 ppm of a yeast extract as defined according to any of claims 1 to 7 into said product.

12. The masking method according to claim 11, **characterised in that** incorporating the yeast extract into the product is carried out during or after the manufacture of said product.
